(19) Europäisches Patentamt
European Patent Office
Office européen des brevets

(11) Veröffentlichungsnummer : **0 071 572**
**B1**

(12) **EUROPÄISCHE PATENTSCHRIFT**

(45) Veröffentlichungstag der Patentschrift : **28.08.85**

(51) Int. Cl.⁴ : **C 07 F 9/58**, C 07 D213/64

(21) Anmeldenummer : **82810313.5**

(22) Anmeldetag : **22.07.82**

(54) **Derivate der 2-Nitro-4- oder 5-Pyridyloxy-phenylphosphonsäure, Verfahren zu ihrer Herstellung, ihre Verwendung als Herbizide und/oder Pflanzenwachstumsregulatoren und/oder Mikrobizide, sowie zur Herstellung der Derivate verwendete Zwischenprodukte, Verfahren zu deren Herstellung und deren Verwendung als Herbizide.**

(30) Priorität : **28.07.81 CH 4891/81**

(43) Veröffentlichungstag der Anmeldung : **09.02.83 Patentblatt 83/06**

(45) Bekanntmachung des Hinweises auf die Patenterteilung : **28.08.85 Patentblatt 85/35**

(84) Benannte Vertragsstaaten : **AT BE CH DE FR GB IT LI NL**

(56) Entgegenhaltungen :
**EP-A- 0 014 684**
**EP-A- 0 024 259**
**US-A- 4 132 783**

(73) Patentinhaber : **CIBA-GEIGY AG**
**Postfach**
**CH-4002 Basel (CH)**

(72) Erfinder : **Maier, Ludwig, Dr.**
**Im Lee 28**
**CH-4144 Arlesheim (CH)**
Erfinder : **Rempfler, Hermann, Dr.**
**Brücklismattstrasse 16**
**CH-4107 Ettingen (CH)**
Erfinder : **Dürr, Dieter, Dr.**
**Brändelistalweg 16**
**CH-4103 Bottmingen (CH)**

**EP 0 071 572 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Derivate der 2-Nitro-4- oder -5-pyridyloxy-phenylphosphonsäure der Formel I, die Herstellung dieser Verbindungen, ferner pflanzenwachstumsregulierende und/oder herbizide und/oder mikrobizide Mittel, welche als Aktivsubstanzen eine Verbindung der Formel I enthalten, und die Verwendung von Verbindungen der Formel I oder sie enthaltender Mittel zur Regulierung des Pflanzenwachstums, zur Bekämpfung von Unkräutern und/oder zur Bekämpfung phytopathogener Mikroorganismen.

Die Erfindung betrifft ferner neue, zur Herstellung von Verbindungen der Formel I verwendete Zwischenprodukte der Formel II, ihre Herstellung, herbizide Mittel, welche als Aktivsubstanzen eine Verbindung der Formel II enthalten, und die Verwendung von Verbindungen der Formel II oder sie enthaltender Mittel zur Bekämpfung von Unkräutern.

In der europäischen Patentanmeldung 14684 sind herbizid wirksame und das Pflanzenwachstum hemmende 2-substituierte 5-Phenoxy-phenylphosphonsäurederivate beschrieben worden. Pyridyloxyphenoxy-alkancarbonsäurederivate mit herbizider und das Pflanzenwachstum beeinflussender Wirkung sind aus der europäischen Patentanmeldung 176 bekannt. Ferner sind in der US-PS 4,235,621 2-substituierte Phenoxy-3-chlor-5-trifluormethyl-pyridine mit herbizider Wirkung beschrieben worden.

Aus der europäischen Patentanmeldung 24259 sind herbizid wirksame Derivate der 5-(Pyridyl-2'-oxy)-2-nitrobenzoesäure bekannt.

Ferner beschreibt die europäische Patentanmeldung 65374 einige 4-(subst. 2-Pyridyloxy)-1,2-dinitrobenzole, und aus der japanischen Patentpublikation 56-86162 ist 4-(3,5-Dichlor-pyridyl-2-oxy)-1,2-dinitrobenzol bekannt.

Die erfindungsgemässen Derivate der 2-Nitro-4- oder -5-pyridyloxyphenylphosphonsäure entsprechen der Formel I

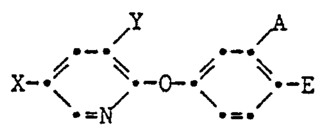

(I)

worin X Halogen oder Trifluormethyl, Y Wasserstoff oder Halogen, einer der beiden Substituenten A und E eine Phosphonatgruppe

$$-\overset{O}{\overset{\|}{P}}(OR)_2$$

worin R für $C_1$-$C_4$-Alkyl oder Wasserstoff steht, und der andere eine Nitrogruppe bedeutet, unter Einschluss ihrer Metallsalze und ihrer Salze mit Ammonium und organischen Stickstoffbasen.

Die Aktivstoffe der Formel I umfassen somit freie Säuren, Ester und Salze.

Unter Halogen sind Fluor, Brom, Jod und insbesondere Chlor zu verstehen.

Als $C_1$-$C_4$-Alkyl kommen Methyl, Aethyl, n-Propyl, Isopropyl, primäres, sekundäres, tertiäres Butyl und Isobutyl in Betracht, vorzugsweise Methyl und Aethyl.

Als zur Salzbildung geeignete Metalle kommen Erdalkalimetalle, wie Magnesium oder Calcium, vor allem aber die Alkalimetalle in Betracht, wie Lithium, Kalium und insbesondere Natrium. Ferner sind als Salzbildner auch Uebergangsmetalle wie beispielsweise Eisen, Nickel, Kobalt, Kupfer, Zink, Chrom oder Mangan geeignet.

Beispiele für zur Salzbildung geeignete organische Stickstoffbasen sind primäre, sekundäre und tertiäre, aliphatische und aromatische, gegebenenfalls am Kohlenwasserstoffrest hydroxylierte Amine, wie Methylamin, Aethylamin, Propylamin, Isopropylamin, die vier isomeren Butylamine, Dimethylamin, Diäthylamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin, Isochinolin sowie Methanolamin, Aethanolamin, Propanolamin, Dimethanolamin, Diäthanolamin oder Triäthanolamin. Als organische Stickstoffbasen kommen auch quaternäre Ammoniumbasen in Betracht.

Beispiele für quaternäre Ammoniumbasen sind das Ammoniumkation, Tetraalkylammoniumkationen, in denen die Alkylreste unabhängig voneinander geradkettige oder verzweigte $C_1$-$C_6$-Alkylgruppen sind, wie das Tetramethylammoniumkation, das Tetraäthylammoniumkation oder das Trimethyläthylammoniumkation, sowie weiterhin das Trimethylbenzylammoniumkation, das Triäthylbenzylammoniumkation und das Trimethyl-2-hydroxyäthylammoniumkation.

Von den Verbindungen der Formel I sind diejenigen bevorzugt, in denen X für Chlor oder Trifluormethyl, Y für Wasserstoff oder Chlor, einer der Substituenten A und E für die Phosphonatgruppe

$$-\overset{O}{\overset{\|}{P}}(OR)_2$$

in welcher R Wasserstoff, Methyl oder Aethyl bedeutet, und der andere für die Nitrogruppe steht.

Von besonderem Interesse sind Verbindungen der Formel I, in denen A für die Phosphonatgruppe

$$-\overset{O}{\overset{\|}{P}}(OR)_2$$

in welcher R Wasserstoff, Methyl oder Aethyl bedeutet, E für die Nitrogruppe steht, und X und Y die für Formel I angegebenen Bedeutungen haben.

Besonders bevorzugt sind Verbindungen der Formel I, in denen A für die Phosphonatgruppe

$$-\overset{O}{\overset{\|}{P}}(OR)_2$$

in welcher R Wasserstoff, Methyl oder Aethyl bedeutet, E für die Nitrogruppe steht, und X Chlor oder Trifluormethyl und Y Wasserstoff oder Chlor bedeutet.

Speziell bevorzugte Verbindungen sind

2-Nitro-5-(5′-trifluormethyl-2′-pyridyloxy)-phenylphosphonsäure-dimethylester ;
2-Nitro-5-(5′-trifluormethyl-2′-pyridyloxy)-phenylphosphonsäure ;
2-Nitro-5-(3′-5′-dichlor-2′-pyridyloxy)-phenylphosphonsäure-dimethylester ;
2-Nitro-5-(3′,5′-dichlor-2′-pyridyloxy)-phenylphosphonsäure ;
2-Nitro-5-(3′-chlor-5′-trifluormehtyl-2′-pyridyloxy)-phenylphosphonsäure-dimethylester ;
2-Nitro-5-(3′-chlor-5′-trifluormethyl-2′-pyridyloxy)-phenylphosphonsäure-diäthylester ;
2-Nitro-5-(3′-chlor-5′-trifluormethyl-2′-pyridyloxy)-phenylphosphonsäure ;
2-Nitro-5-(3′,5′-dichlor-2′-pyridyloxy)-phenylphosponsäurediäthylester ;
2-Nitro-5-(5′-trifluormethyl-2′-pyridyloxy)-phenylphosphonsäurediäthylester ; und
2-Nitro-4-(5′-trifluormethyl-2′-pyridyloxy)-phenylphosphonsäure-dimethylester.

Die Herstellung der neuen Phosphonsäurederivate der Formel I erfolgt in Analogie zur bekannten Herstellung von 2-Nitro-phenylphosphonsäuren (J. Chem. Soc. (C), *1969,* 1314), indem man ein 1,2-Dinitro-4-pyridyloxybenzol der Formel II

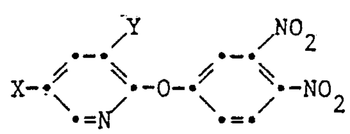

(II)

worin X Halogen oder Trifluormethyl und Y Wasserstoff oder Halogen bedeutet, mit einem Trialkylphosphit der Formel III

$$P(OR')_3 \qquad (III)$$

worin R′ für $C_1$-$C_4$-Alkyl steht, umsetzt, und gewünschtenfalls den erhaltenen Phosphonsäuredialkylester der Formel IV

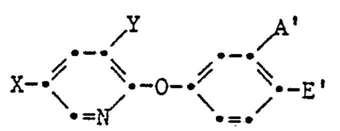

(IV)

worin X und Y die für Formel II angegebenen Bedeutungen haben, einer der beiden Subsituenten A′ und E′ eine Phosphonatgruppe

$$-\overset{O}{\overset{\|}{P}}(OR')_2$$

worin R′ die vorstehend angegebene Bedeutung hat, und der andere eine Nitrogruppe darstellt, in an sich bekannter Weise in die entsprechende freie Phosphonsäure, ein Metallsalz oder eine Salz mit Ammonium oder einer organischen Stickstoffbase überführt.

Die Umsetzung einer Verbindung der Formel II mit einer Verbindung der Formel III erfolgt zweckmässigerweise bei einer Temperatur zwischen 50° und 150 °C, vorzugsweise zwischen 70° und 120 °C. Die Umsetzung lässt sich ohne Verwendung eines Lösungsmittels durchführen, wird jedoch vorzugsweise in einem organischen, aprotischen Lösungsmittel, wie beispeilsweise Acetonitril, Benzol oder Toluol, durchgeführt.

Die Phosphonatestergruppe lässt sich beispielsweise durch Behandeln mit 2 Mol $SOCl_2$ in

Gegenwart von Dimethylformamid als Katalysator bei erhöhter Temperatur gemäss dem in der US-PS 4.213.922 beschriebenen Verfahren leicht in das Phosphonsäuredichlorid überführen, welches durch Hydrolyse in die freie Phosphonsäure übergeführt werden kann.

Die freie Phosphonsäure lässt sich auch herstellen, indem man das Dialkylphosphonat mit konzentrierter Salzsäure behandelt oder indem man es mit $(CH_3)_3SiBr$ umsetzt und die erhaltene Silylverbindung hydrolysiert.

Die Zwischenprodukte der Formel II

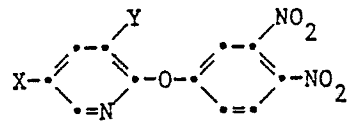

(II)

worin X Halogen oder Trifluormethyl und Y Wasserstoff oder Halogen bedeutet mit der Massgabe, dass X nicht Chlor oder Trifluormethyl bedeutet, wenn Y für Wasserstoff oder Chlor steht, die speziell für die Herstellung der erfindungsgemässen Verbindungen der Formel I entwickelt wurden, sind noch neu und bilden ebenfalls einen Gegenstand der Erfindung.

Die Herstellung der Verbindungen der Formel II erfolgt, indem man eine Verbindung der Formel V

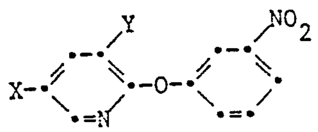

(V)

worin X Halogen oder Trifluormethyl und Y Wasserstoff oder Halogen bedeutet, mit einem Nitriersäuregemisch nitriert. Als Nitriersäuregemisch kommen übliche Zusammensetzungen wie beispielsweise konzentrierte Schwefelsäure und Alkalinitratsalze in Betracht. Die Nitrierung verläuft exotherm und wird zweckmässigerweise bei Normaldruck unter Kühlung oder höchtens bei Raumtemperatur vorgenommen.

Die Ausgangsprodukte der Formel V lassen sich in Analogie zur bekannten Herstellung von 3-Nitrodiphenyläther (europäische Patentanmeldung 7471) herstellen, indem man eine Verbindung der Formel VI

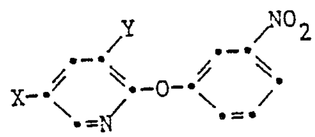

(VI)

worin X Halogen oder Trifluormethyl, Y Wasserstoff oder Halogen und Hal Halogen bedeutet, in Gegenwart einer Base und vorzugsweise in einem inerten organischen Lösungsmittel mit meta-Nitrophenol umsetzt. Die Umsetzung kann ohne oder in Anwesenheit von gegenüber den Reaktionsteilnehmern inerten Lösungs- oder Verdünnungsmitteln durchgeführt werden. Bevorzugt sind polare organische Lösungsmittel wie Methyläthylketon, Dimethylformamid, Dimethylsulfoxid. Die Reaktionstemperaturen liegen zwischen 0 und 200 °C, vorzugsweise zwichen 20 und 100 °C, und die Reaktionsdauer beträgt je nach Ausgangsstoff, gewählter Umsetsungstemperatur und Lösungsmittel etwa 1 Stunde bis mehrere Tage. Die Umsetzung erfolgt in der Regel bei Normaldruck. Als Basen lassen sich anorganische oder organische Basen, wie beispielsweise KOH, $NaOCH_3$, $NaHCO_3$, $K_2CO_3$, Kalium-tert. butylat oder Triäthylamin verwenden.

Die Ausgangsprodukte der Formel VI sind bekannt oder lassen sich analog bekannten Verfahren herstellen.

Zur Applikation als Herbizide, Pflanzenwuchsregulatoren oder Mikrobizide werden die Verbindungen der Formel I in unveränderter Form oder vorzugsweise zusammen mit den in der Formulierngstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie

Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimstein, Ziegelbruch, Sepiolit oder Bentonit, als nicht sorptive Trägermaterialien z. B. Calcit oder Sand in Frage. Darüberhinaus können eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen genannt, die z. B. aus Kokosnuss- oder Talgöl gewonnen werden können. Ferner sind auch die Fettsäuremethyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst. z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlösliche, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthenol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfatge oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben :

« Mc Cutcheon's Detergents and Emulsifiers Annual » Mc
Publishing Corp., Ridgewood New Jersey, 1980
Sisely and Wood, « Encyclopedia of Surface Active Agents »,
Chemical Publishing Co., Inc. New York, 1980.

Die agrochemischen Zubereitungen enthalten in der Regel 0,1 bis 99 %, insbesondere 0,1 bis 95 %, Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die vorstehenden Ausführungen über Applikationsformen und -möglichkeiten gelten ebenso wie die nachfolgenden Formulierungsbeispiele auch für Verbindungen der Formel II.

Die Verbindungen der Formeln I und II weisen herbizide Wirksamkeit auf und sind zur pre- und postemergenten Bekämpfung mono- und dicotyler Unkräuter geeignet.

Die Verbindungen der Formel I zeichnen sich zudem dadurch aus, dass sie pflanzenwachstumsregulierende Eigenschaften besitzen. Nach bisherigen Erfahrungen gilt für die Applikation von Pflanzenwachstumsregulatoren, dass die Aktivsubstanzen eine oder mehrere unterschiedliche Wirkungen bei den Pflanzen hervorrufen können. Diese verschiedenartigen Wirkungen hängen im wesentlichen vom Zeitpunkt der Anwendung, d. h., vom Entwicklungsstadium des Samens oder der Pflanze, der Art der Applikation sowie von den angewendeten Konzentrationen ab. Derartige Effekte sind aber wiederum je nach Pflanzenart verschieden. Die Applikation von Verbindungen der Formel I eröffnet somit die Möglichkeit, das Wachstum von Pflanzen in gewünschter Weise zu beeinflussen.

Mit den neuen Wirkstoffen der Formel I lässt sich beispielsweise das Pflanzenwachstum dahingehend regulieren, dass das generative Wachstum der Pflanzen gefördert und damit eine Ertragssteigerung erzielt wird. Als Kulturpflanzen, bei denen der Ernteertrag erheblich gesteigert werden kann, kommen insbesondere Leguminosen wie Bohnen, Erbsen, Linsen und vor allem Soja in Betracht. Die Ertragssteigerung kann auf einer Erhöhung der Anzahl der Schoten und/oder ihres Gewichts beruhen.

Die neuen Verbindungen der Formel I fördern ferner das Wurzelwachstum bei Kulturpflanzen, vor allem bei Baumwolle und Getreide, insbesondere bei Weizen. Daraus resultiert eine bessere Wasser- und Nährstoffversorgung und ein erhöhter Schutz von Getreidekulturen gegen Lagerung durch Sturm oder Unwetter.

Die Verbindungen der Formel I sind zudem mikrobizid wirksam und eignen sich zur Bekämpfung phytopathogener Pilze oder Bakterien.

Die Herstellung von Zwischenprodukten der Formel II wird durch die nachfolgend angegebene Arbeitsweise näher veranschaulicht :

25 g 3-(3′,5′-dichlor-2′-pyridyloxy)-nitrobenzol werden in 100 ml konzentrierter Schwefelsäure und 25 ml Aethylenchlorid vorgelegt. Bei 10 bis 15 °C wird während 30 Minuten eine Lösung von 5,7 g rauchender Salpetersäure in 8,8 g konzentrierter Schwefelsäure zugetropft. Anschliessend wird 2 Stunden lang bei Raumtemperatur gerührt und dann das Reaktionsgemisch auf Eis gegossen. Die Ausgefallene kristalline Masse wird abfiltriert, mit Wasser gewaschen und aus Alkohol umkristallisiert. Man erhält 18,5 g 1,2-Dinitro-4-(3′,5′-dichlor-2′-pyridyloxy)-benzol.

Smp. 163-165 °C.

Auf analoge Weise können auch folgende Verbindungen der Formel II hergestellt werden :

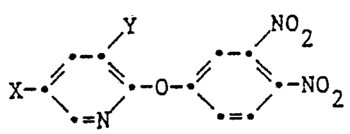

Tabelle 1

| Nr. | X | Y | Smp. |
|---|---|---|---|
| II-2 | $CF_3$ | Cl | 95-98°C |
| II-3 | $CF_3$ | H | 82-85°C |

Herstellungsbeispiele für Wirkstoffe der Formel I

Beispiel 1

2-Nitro-5-(5′-trifluormethyl-2′-pyridyloxy)-phenylphosphonsäure-dimethylester (Verbindung Nr. 1)

Eine Mischung aus 24,8 g 1,2-Dinitro-4-(5′-trifluormethyl-2′-pyridyloxy)-benzol und 10,7 ml Trimethylphosphit in 100 ml Toluol wird solange unter Rühren zum Rückfluss erhitzt, bis sich kein Methylnitrit mehr bildet (ca. 40 Stunden). Die erhaltene dunkel gefährbte Lösung wird am Rotationsverdampfer eingedampft und über Silicagel mit Essigester als Laufmittel chromatographiert. Man erhält zuerst 8 g (27,1 % d. Th.) 2-Nitro-5-(5′-trifluormethyl-2′-pyridyloxy)-phenylphosphonsäure-dimethylester als orangefarbenes Oel. $n_D^{20}$ 1 531 5.

Analyse des Endproduktes $C_{14}H_{12}F_3N_2O_6P$ (393,2) :
berechnet : C 42,87 H 3,09 N 7,14 F 14,53 %
gefunden : C 41,50 H 3,23 N 6,86 F 14,11 %
$^1$H-NMR (in $CDCl_3$) : $OCH_3$ 3,9 ppm ($J_{POCH}$ = 11 Hz, 6H) ; aromat. CH von 7,15-8,6 (m, 6H).

Als weitere Fraktion werden auch 1,5 g (5,1 % d. Th.) 2-Nitro-4-(5'-trifluormethyl-2'-pyridyloxy)-phenylphosphonsäure-dimethylester als orangefarbenes Oel erhalten (Verbindung Nr. 2). $^1$H-NMR (in $CDCl_3$) zeigt bei 8,1 ppm ein Proton, das mit einem anderen Proton koppelt und zusätzlich eine $J_{PCH}$ = 13-14 Hz aufweist.

Auf analoge Weise erhält man 2-Nitro-5-(5'-trifluormethyl-2'-pyridyloxy)-phenylphosphonsäurediäthylester (Verb. Nr. 10), Smp. 75-76 °C.

## Beispiel 2

2-Nitro-5-(5'-trifluormethyl-2'-pyridyloxy)-phenylphosphonsäure (Verbindung Nr. 3)

Eine Mischung aus 7,0 g 2-Nitro-5-(5'-trifluormethyl-2'-pyridyloxy)-phenylphosphonsäure-dimethylester und 6 ml $(CH_3)_3SiBr$ wird 2 Tage bei Raumtemperatur gehalten, dann in Aethanol eingegossen und am Rotationsverdampfer eingedampft. Der Rückstand wird in Aethanol gelöst (50 ml) und mit 100 ml Wasser versetzt. Die auskristallisierte 2-Nitro-5-(5'-trifluormethyl-2'-pyridyloxy)-phenylphosphonsäure (3,8 g = 58,6 % d. Th.) wird abfiltriert und bei 80 °C im Vakuum getrocknet. Smp. 217-219 °C (schwarze Schmelze).

Analyse des Endproduktes $C_{12}H_8F_3N_2O_6P$ (364,1) :
berechnet : C 39,58 H 2,22 N 7,69 F 15,65 %
gefunden : C 39,64 H 2,31 N 7,81 F 15,64 %
$^1$H-NMR (in $CD_3OD$) : OH 5,2 ppm (s, 2H) ; aromat. H 7,2-8,7 (m, 6H).

## Beispiel 3

2-Nitro-5-(3',5'-dichlor-2'-pyridyloxy)-phenylphosphonsäure-dimethylester (Verbindung Nr. 4)

Eine Mischung aus 26,9 g 1,2-Dinitro-4-(3',5'-dichlor-2'-pyridyloxy)-benzol, 11,5 ml Trimethylphosphit und 100 ml Toluol wird unter Rühren 48 Stunden zum Rückfluss erhitzt. Das Reaktionsgemisch wird einge dampft und der Rückstand über Kieselgel (Hesh 60) mit Essigester chromatographiert. Das Eluat wird eingedampft und der Rückstand aus Methylenchlorid/Diisopropyläther umkristallisiert. Man erhält 10,3 g (32,2 % d. Th.) 2-Nitro-5-(3',5'-dichlor-2'-pyridyloxy)-phenylphosphonsäure-dimethylester in Form gelber Kristalle. Smp. 101-103 °C.

Analyse des Endproduktes $C_{13}H_{11}Cl_2N_2O_6P$ (393,1) :
berechnet : C 39,72 H 2,82 N 7,13 Cl 18,04 %
gefunden : C 39,87 H 2,94 N 7,18 Cl 17,96 %
$^1$H-NMR (in $CDCl_3$) : $OCH_3$ 3,9 ppm (d,$J_{POCH}$ = 11 Hz, 6H) ; aromat. H 7,3-8,3 ppm (5H).

Auf analoge Weise erhält man 2-Nitro-5-(3',5'-dichlor-2'-pyridyloxy)-phenylphosphonsäurediäthylester als braunes, viskoses Oel (Verb. Nr. 9).

## Beispiel 4

2-Nitro-5-(3',5'-dichlor-2'-pyridyloxy)-phenylphosphonsäure (Verbindung Nr. 5)

Eine Mischung aus 6,15 g 2-Nitro-5-(3',5'-dichlor-2'-pyridyloxy)-phosphonsäure-dimethylester und 10 ml $(CH_3)_3SiBr$ wird 20 Stunden bei Raumtemperatur gerührt. Anschliessend wird Alkohol zugesetzt und am Rotationsverdampfer eingedampft. man erhält 4,9 g 2-Nitro-5-(3',5'-dichlor-2'-pyridyloxy)-phenylphosphonsäure in Form orangefarbener Kristalle. Smp. 238 °C (Zersetzung).

Analyse des Endproduktes $C_{11}H_7Cl_2N_2O_6P$ (365,0) :
berechnet : C 36,19 H 1,93 N 7,67 Cl 19,42 %
gefunden : C 34,92 H 1,97 N 7,36 Cl 19,36 %
$^1$H-NMR (in $CD_3OD/D_2O$) : OH 5,1 ppm (br., 2H) ; aromat. H 7,2-8,6 ppm (br., 5H).

## Beispiel 5

2-Nitro-5-(3'-chlor-5'-trifluormethyl-2'-pyridyloxy)-phenylphosphonsäure-dimethylester (Verbindung Nr. 6)

Eine Mischung aus 10 g 1,2-Dinitro-4-(3'-chlor-5'-trifluormethyl-2'-pyridyloxy)-benzol, 6,4 ml Trimethylphosphit und 35 ml Toluol wird unter Rühren 18 Stunden zum Rückfluss erhitzt, mit Aktivkohle versetzt und eingedampft. Man erhält ein gelbes Oel, das nach chromatographischer Reinigung (Kieselgel ; Essigester als Laufmittel) 4,42 g (38,3 % d. Th.) 2-Nitro-5-(3'-chlor-5'-trifluormethyl-2'-pyridyloxy)-phenylphosphonsäure-dimethylester in Form gelblicher Kristalle ergibt. Smp. 99-100 °C.

Analyse des Endproduktes $C_{14}H_{11}ClF_3N_2O_6P$ (426,6) :
berechnet : C 39,41 H 2,60 N 6,57 %
gefunden : C 39,33 H 2,69 N 6,46 %
$^1$H-NMR (in $CDCl_3$) : $OCH_3$ 3,9 ppm (d,$J_{POCH}$ = 11 Hz, 6H) ; aromat. H 7,5-8,4 ppm (m, 5H).

## Beispiel 6

2-Nitro-5-(3'-chlor-5'-trifluormethyl-2'-pyridyloxy)-phenylphosphonsäure-diäthylester (Verbindung Nr. 7)

Analog dem in Beispiel 5 beschriebenen Verfahren werden ausgehend von 10 g 1,2-Dinitro-4-(3'-chlor-5'-trifluormethyl-2'-pyridyloxy)-benzol, 8,6 ml Triäthylphosphit und 35 ml Toluol unter 16 stündigem Erhitzen zum Rückfluss und chromatographischer Reinigung 7,8 g (63,5 % d.Th.) 2-Nitro-5-(3'-chlor-5'-trifluormethyl-2'-pyridyloxy)-phenylphosphonsäure-diäthylester in Form eines hochviskosen Oels hergestellt.
Analyse des Endproduktes $C_{16}H_{15}ClF_3N_2O_6P$ (454,7) :
berechnet : C 42,26 H 3,32 N 6,16 Cl 7,80 P 6,81 %
gefunden : C 42,25 H 3,52 N 6,17 Cl 7,89 P 6,61 %
$^1$H-NMR (in $CDCl_3$) : $CH_3$ 1,37 ppm (t, 6H) ; $OCH_2$ 4,2 ppm (qui, 4H) ; aromat. H 7,35-8,4 (m, 5H).

## Beispiel 7

2-Nitro-5-(3'-chlor-5'-trifluormethyl-2'-pyridyloxy)-phenylphosphonsäure (Verbindung Nr. 8)

Eine Mischung aus 3,5 g 2-Nitro-5-(3'-chlor-5'-trifluormethyl-2'-pyridyloxy)-phenylphosponsäurediäthylester und 50 ml HCl (20 %ig) wird 14 Stunden zum Rückfluss erhitzt und anschliessend am Rotationsverdampfer eingedampft. Man erhält 2,54 g (82,7 % d. Th.) 2-Nitro-5-(3'-chlor-5'-trifluormethyl-2'-pyridyloxy)-phenylphosphonsäure. Smp. 156-159 °C.
$^1$H-NMR (in $D_2O$/NaOD) : OH 4,75 (s, 2H) ; aromat. H 6,2-8,1 (m, 5H).

Formulierungsbeispiele für flüssige Wirkstoffe der Formel I oder II (% = Gewichtsprozent)

| 8. Emulsions-Konzentrate | a) | b) | c) |
|---|---|---|---|
| Wirkstoff aus Beispiel 1-8 oder Tabelle 1 | 25 % | 40 % | 50 % |
| Ca-Dodecylbenzolsulfonat | 5 % | 8 % | 6 % |
| Ricinusölpolyäthylenglykoläther (36 Mol AeO) | 5 % | — | — |
| Tributylphenolpolyäthylenglykoläther (30 Mol AeO) | — | 12 % | 4 % |
| Cyclohexanon | — | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 9. Lösungen | a) | b) | c) | d) |
|---|---|---|---|---|
| Wirkstoff aus Beispiel 1-8 oder Tabelle 1 | 80 % | 10 % | 5 % | 95 % |
| Aethylenglykolmonomethyläther | 20 % | — | — | — |
| Polyäthylenglykol MG 400 | — | 70 % | — | — |
| N-Methyl-2-pyrrolidon | — | 20 % | — | — |
| Epoxydiertes Kokosnussöl | — | — | 1 % | 5 % |
| Benzin (Siedegrenzen 160-190 °C) | — | — | 94 % | — |

Die Lösungen sind zur Anwendung in Form kleinster Tropfen geeignet.

| 10. Granulate | a) | b) |
|---|---|---|
| Wirkstoff aus Beispiel 1-8 oder Tabelle 1 | 5 % | 10 % |
| Kaolin | 94 % | — |
| Hochdisperse Kieselsäure | 1 % | — |
| Attapulgit | — | 90 % |

Der Wirkstoff wird in Methylenchlorid gelöst, auf den Träger aufgesprüht und das Lösungsmittel anschliessend in Vakuum abgedampft.

11. Stäubemittel

| | a) | b) |
|---|---|---|
| Wirkstoff aus Beispiel 1-8 oder Tabelle 1 | 2 % | 5 % |
| Hochdisperse Kieselsäure | 1 % | 5 % |
| Talkum | 97 % | — |
| Kaolin | — | 90 % |

Durch inniges Vermischen der Trägerstoffe mit dem Wirkstoff erhält man gebrauchsfertige Stäubemittel.

Formulierungsbeispiele für feste Wirkstoffe der Formel I oder II (% = Gewichtsprozent)

12. Spritzpulver

| | a) | b) | c) |
|---|---|---|---|
| Wirkstoff aus Beispiel 1-8 oder Tabelle 1 | 25 % | 50 % | 75 % |
| Na-Ligninsulfonat | 5 % | 5 % | — |
| Na-Laurylsulfat | 3 % | — | 5 % |
| Na-Diisobutylnaphthalinsulfonat | — | 6 % | 10 % |
| Octylphenolpolyäthylenglykoläther (7-8 Mol AeO) | — | 2 % | — |
| Hochdisperse Kieselsäure | 5 % | 10 % | 10 % |
| Kaolin | 62 % | 27 % | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

13. Emulsions-Konzentrat

| | |
|---|---|
| Wirkstoff aus Beispiel 1-8 oder Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

14. Stäubemittel

| | a) | b) |
|---|---|---|
| Wirkstoff aus Beispiel 1-8 oder Tabelle 1 | 5 % | 8 % |
| Talkum | 95 % | — |
| Kaolin | — | 92 % |

Man erhält anwendungsfertige Stäubemittel, indem der Wirkstoff mit den Trägerstoffen vermischt und auf einer geeigneten Mühle vermahlen wird.

15. Extruder-Granulat

| | |
|---|---|
| Wirkstoff aus Beispiel 1-8 oder Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

16. Umhüllungs-Granulat

| | |
|---|---|
| Wirkstoff aus Beispiel 1-8 oder Tabelle 1 | 3 % |
| Polyäthylenglykol (MG200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

17. Suspensions-Konzentrat

| | |
|---|---|
| Wirkstoff aus Beispiel 1-8 oder Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält son ein Suspensions-Konzentrat, aus welchem durch Verdünnen mit Wasser Suspensionen jeder gewünschten Konzentration hergestellt werden können.

## Biologische Beispiele

### Beispiel 18

Pre-emergente Herbizid-Wirkung (Keimhemmung)

Im Gewächshaus wird unmittelbar nach Einsaat der Samen von Versuchspflanzen in Saatschalen die Erdoberfläche mit einer wässrigen Dispersion von Wirkstoffen der Formel I, erhalten aus 10 %igem Spritzpulver, behandelt. Es werden Konzentrationen von 4 kg Wirksubstanz pro Hektar angewendet. Die Saatschalen werden im Gewächshaus bei 22-25 °C und 50-70 % relativer Luftfeuchtigkeit gehalten. Der Versuch wird nach 3 Wochen ausgewertet und die Resultate werden nach folgender Notenskala bonitiert :

1 = Pflanzen nicht gekeimt oder total abgestorben
2-3 = sehr starke Wirkung
4-6 = mittlere Wirkung
7-8 = geringe Wirkung
9 = keine Wirkung (wie unbehandelte Kontrolle)

Von den Verbindungen der Formel I zeigte in diesem Test insbesondere 2-Nitro-5-(3′-chlor-5′-trifluormethyl-2′-pyridyloxy)-phenylphosponsäure-dimethylester (Verbindung Nr. 6) eine ausgezeichnete Wirkung gegen die Unkräuter Setaria und Stellaria (Note 1) und Sinapis (Note 2).

### Beispiel 19

Post-emergente Herbizid-Wirkung (Kontaktherbizid)

Die zu testenden Unkräuter werden nach dem Auflaufen (im 4- bis 6-Blattstadium) mit einer wässrigen Dispersion von Wirkstoffen der Formel I, erhalten aus a) einem 10 %igen oder b) einem 25 %igen Spritzpulver oder c) einem 25 %igen Emulsionskonzentrat, in einer Dosierung von 4 kg Wirkstoff pro Hektar auf die Pflanzen gespritzt und diese bei 24-26 °C und 45-60 % relativer Luftfeuchtigkeit gehalten. Nach 15 Tagen wird der Versuch ausgewertet und das Ergebnis nach derselben Notenskala wie im preemergenten Versuch (Beispiel 19) bonitiert.

Von den Verbindungen der Formel I erwiesen sich vor allem 2-Nitro-5-(3′-chlor-5′-trifluormethyl-2′-pyridyloxy)-phenylphosphonsäure-dimethylester (Verbindung Nr. 6), 2-Nitro-5-(3′.5′-dichlor-2′-pyridyloxy)-phosphonsäure-dimethylester (Verbindung Nr. 4), 2-Nitro-5-(5′-trifluormethyl-2′-pyridyloxy)-phenylphosphonsäure-dimethylester (Verbindung Nr. 1), 2-Nitro-5-(3′-chlor-5′-trifluormethyl-2′-pyridyloxy)-phenylphosphonsäure-diäthylester (Verb. Nr. 7) und 2-Nitro-5-(3′,5′-dichlor-2′-pyridyloxy)-phenylphosphonsäure-diäthylester (Verb. Nr. 9), als gut wirksam :

Tabelle 2

| Verbindung Nr. | Formulierung | Testpflanze Solanum | Sinapis | Setaria |
|---|---|---|---|---|
| 6 | a) | 1 | 1 | 3 |
| 4 | b) | 2 | 3 | 3 |
| 1 | c) | 4 | 3 | 4 |
| 7 | c) | 1 | 2 | 3 |
| 9 | c) | 1 | 2 | 2 |

Beispiel 20

Regulierung des Wachstums von Sojapflanzen

a) In Kunststoffbehälter mit einem Gemisch aus Erde, Torf und Sand im Verhältnis 6 : 3 : 1 werden Sojabohnen der Sorte « Hark » eingesät und in einer Klimakammer unter kontrollierten Bedingungen gehalten. Durch Einstellen optimaler Bedingungen bezüglich Temperatur, Beleuchtung, Düngung und Bewässerung entwickeln sich die Pflanzen in ca. 5 Wochen zum 5-6-Trifolia-Blattstadium. Zu diesem Zeitpunkt werden die Pflanzen mit einer wässrigen Brühe eines Wirkstoffs der Formel I, erhalten aus einem 25 %igen Emulsionskonzentrat, bis zur Benetzung in Aufwandmengen von 0,1 kg, 0,5 kg und 1,5 kg Wirkstoff pro Hektar besprüht. Die Auswertung erfolgt ca. 5 Wochen nach Applikation des Wirkstoffs, wobei die mittlere Wuchshöhe und die Erträge der geernteten Schoten mit denen der unbehandelten Kontrolle (= 100 %) verglichen werden.

Von den Verbindungen der Formel I erwies sich in diesem Test insbesondere 2-Nitro-5-(5'-trifluormethyl-2'-pyridyloxy)-phenylphosphonsäure -dimethylester (Verbindung Nr. 1) als sehr wirksam ;

b) unter gleichen Versuchsbedingungen wie unter a) angegeben, jedoch mit Aufwandmengen von 0,08 bis 4,0 kg Wirksubstanz pro Hektar werden beispielsweise für 2-Nitro-5-(5'-trifluormethyl-2'-pyridyloxy)-phenylphosphonsäure (Verbindung Nr. 3) folgende Resultate erhalten :

Tabelle 3

| kg Wirkstoff/ha | 0,08 | 0,40 | 0,80 | 4,00 |
|---|---|---|---|---|
| Anzahl der Schoten in % der Kontrolle | 107 | 119 | 109 | 98 |
| Gewicht der Schoten in % der Kontrolle | 119 | 119 | 135 | 108 |

(Kontrolle = 100 %)

Beispiel 21

Förderung des Wurzelwachstums bei Weizen

Verbindungen der Formel I werden als wässrige Dispersionen, erhalten aus 25 %igem Spritzpulver, angewendet. Der Versuch wird mit Samen durchgeführt, die in mit Erde gefüllte Plastikzylinder von 5 × 30 cm eingesät werden (10 Samen pro Zylinder), wobei a) die Samen vor der Einsaat in Aufwandmengen von 4-130 mg pro kg Saatgut behandelt werden oder b) unbehandelte Samen eingesät und der Boden mit der Wirkstoffdispersion in Aufwandmengen von 0,3 bis 3 kg pro Hektar besprüht wird. Die Zylinder werden in einer Klimakammer unter kontrollierten Bedingungen gehalten. Nach 10 Tagen werden die Keimlinge durch vorsichtiges Waschen mit Wasser von der Erde befreit und Länge und Trockengewicht der Wurzeln gemessen.

Von den Verbindungen der Formel I zeigte in diesem Test insbesondere 2-Nitro-5-(5'-trifluormethyl-2'-pyridyloxy)-phenylphosphonsäure (Verbindung Nr. 3) eine gute Wirkung.

Beispiel 22

Förderung des Wurzelwachstums bei Weizen und Baumwolle

Verbindungen der Formel I werden als wässrige Dispersionen, erhalten aus 25 %igem Spritzpulver, angewendet. Der Versuch wird mit Samen durchgeführt, die in mit Erde gefüllte Plastikkisten von 5 × 35 × 60 cm eingesät werden (15 Samen pro Kiste), wobei die Samen vor der Einsaat in Aufwandmengen von 45-500 mg pro kg Saatgut behandelt werden. Die Kisten werden in einer Klimakammer unter kontrollierten Bedingungen gehalten. Nach 30 Tagen werden die Pflanzen durch vorsichtiges Waschen mit Wasser von der Erde befreit und Länge und Trockengewicht der Wurzeln gemessen.

Von den Verbindungen der Formel I zeigte in diesem Test insbesondere 2-Nitro-5-(5'-trifluormethyl-2'-pyridyloxy)-phenylphosphonsäure (Verbindung Nr. 3) eine gute Wirkung) :

Tabelle 4

| mg Wirkstoff/kg Samen | 45 | 150 | 500 |
|---|---|---|---|
| Weizen: | | | |
| Länge in % der Kontrolle | 98 | 107 | 107 |
| Gewicht in % der Kontrolle | 105 | 112 | 110 |
| Baumwolle: | | | |
| Länge in % der Kontrolle | 122 | 111 | 122 |
| Gewicht in % der Kontrolle | 105 | 97 | 129 |

(Kontrolle = 100 %)

Beispiel 23

Wirkung gegen Erisyphe graminis auf Gerste

Ca. 8 cm hohe Gerstenpflanzen werden mit einer Spritzbrühe (0,02 % Wirkstoff der Formel I), erhalten aus einem 25 %igen Emulsionskonzentrat, besprüht. Nach 3-4 Stunden werden die behandelten Pflanzen mit Konidien von Erisyphe graminis bestäubt. Die infizierten Gerstenpflanzen werden in einem Gewächshaus bei ca. 22 °C aufgestellt. Die Beurteilung des Pilzbefalls erfolgt nach 10 Tagen.

In diesem Test erwies sich von den Verbindungen der Formel I 2-Nitro-5-(5'-trifluormethyl-2'-pyridyloxy)-phenylphosphonsäure-dimethylester (Verbindung Nr. 1) als besonders vorteilhaft. Der Pilzbefall wurde gegenüber unbehandelten Kontrollpflanzen (= 100 % Befall) um 80-95 % verringert.

Beispiel 24

Wirkung gegen Schimmelpilze an Feucht-Mais

Trockene Maiskörner (Portionen zu je 80 g) werden in verschliessbaren Plastikbechern mit einer wässrigen Suspension eines Wirkstoffs der Formel I, erhalten aus 25 %igem Spritzpulver, gut durchmischt. Die Substanzapplikation wird so bemessen, dass eine Konzentration von 0,06 % Wirkstoff, bezogen auf das Maistrockengewicht, erreicht wird. Ein befeuchteter Papierlappen sorgt für eine feuchtigkeitsgesättigte Atmosphäre in den mit Mais gefüllten und anschliessend verschlossenen Bechern. Nach 2-3 Wochen Inkubation bei ca. 20 °C entwickelt sich bei den nur mit Wasser behandelten Mais-Proben spontan eine Mischpopulation von Schimmelpilzen. Das Ausmass der Pilzentwicklung nach drei Wochen dient zur Beurteilung der Wirksamkeit der Prüfsubstanzen.

Maisproben, die nach 3 Wochen keinen oder nur einen schwachen Pilzbefall aufweisen, werden weitere 2 Monate lang inkubiert. Nach jedem Monat erfolgt eine visuelle Bonitierung nach denselben Kriterien wie vorstehend angegeben.

Von den Verbindungen der Formel I zeigte 2-Nitro-5-(5'-trifluormethyl-idyloxy)-phenylphosphonsäure (Verbindung Nr. 3) eine besonders ausgeprägte fungizide Wirksamkeit. Gegenüber der unbehandelten Kontrolle war der Pilzbefall nach 3 Wochen um 80-95 %, nach einem weiteren Monat ebenfalls um 80-95 % und nach einem weiteren Monat noch um 50-80 % verringert.

Beispiel 25

Wirkung gegen Xanthomonas oryzae auf Reis (Residual-protektive Wirkung)

Reispflanzen der Sorte « Caloro » oder « S6 » werden nach 3-wöchiger Anzucht im Gewächshaus mit einer Spritzbrühe mit verschiedenen Konzentrationen eines Wirkstoffs der Formel I, erhalten aus 10 %igem oder 25 %igem Spritzpulver, besprüht. Nach eintägigem Antrocknen des Spritzbelags werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht wurde, abgeschnitten werden. Nach 10-tägiger Inkubation im Klimaraum werden die angeschnittenen Blätter welk, rollen sich ein und werden nekrotisch. Das Ausmass dieser Krankheitssymptome dient zur Beurteilung der Wirksamkeit der Prüfsubstanzen.

In diesem Test zeigten von den Verbindungen der Formel I vor allem 2-Nitro-5-(3'-chlor-5'-trifluormethyl-2'-pyridyloxy)-phenylphosphonsäure-dimethylester (Verbindung Nr. 6) und 2-Nitro-5-(3',5'-dichlor-2'-pyridyloxy)-phenylphosphonsäure-dimethylester (Verbindung Nr. 4) stark bakterizide Eigenschaften. Mit der Verbindung Nr. 6 wurde bei einer Wirkstoffkonzentration von 0,02 % und mit der Verbindung Nr. 4 bei einer Wirkstoffkonzentration von 0,000 6 % wurde mit der Verbindung Nr. 4 noch eine Wirksamkeit von 80-95 % erreicht.

Beispiel 26

Wirkung gegen Xanthomonas oryzae auf Reis (systemische Wirkung)

Reispflanzen der Sorte « Caloro » oder « S6 » werden nach 3-wöchiger Anzucht im Gewächshaus mit einer Suspension eines Wirkstoffes der Formel I (0,006 und 0,000 6 % Wirkstoff, bezogen auf das Erdvolumen), erhalten aus 25 %igem Spritzpulver, gegossen. Drei Tage nach der Behandlung werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae getaucht wurde, abgeschnitten werden. Nach 10-tägiger Inkubation im Klimaraum werden die angeschnittenen Blätter welk, rollen sich ein und werden nekrotisch. Das Ausmass dieser Krankheitssymptome dient zur Beurteilung der Wirksamkeit der Prüfsubstanzen.

In diesem Test zeigte von den Verbindung der Formel I besonders 2-Nitro-5-(3',5'-dichlor-2'-pyridyloxy)-phenylphosphonsäure-dimethylester (Verbindung Nr. 4) eine ausgeprägte bakterizide Wirksamkeit. So wurde bei einer Wirkstoffkonzentration von 0,006 % eine über 95 %ige, bei einer Wirkstoffkonzentration von 0,000 6 % eine 50-80 %ige Wirkung festgestellt.

Die herbizide Wirkung von Verbindungen der Formel II wird durch folgenden Test näher veranschaulicht :

Die zu testenden Pflanzen werden nach dem Auflaufen (im 4- bis 6-Blattstadium) mit einer wässrigen Dispersion eines Wirkstoffs der Formel II, erhalten aus einem 25 %igen spritzpulver, in einer Konzentration von 4 kg Wirkstoff pro Hektar auf die Pflanzen gespritzt und diese bei 24-26 °C und 45-60 % relativer Luftfeuchtigkeit gehalten. Nach 15 Tagen wird der Versuch ausgewertet und das Ergebnis nach folgender Notenskala bonitiert :

1 = Pflanzen total abgestorben
2-3 = sehr starke Wirkung
4-6 = mittlere Wirkung
7-8 = geringe Wirkung
9 = keine Wirkung (wie unbehandelte Kontrolle)

Von den Verbindungen der Formel II zeigten in diesem Test 1,2-Dinitro-4-(3'-chlor-5'-trifluormethyl-2'-pyridyloxy)-benzol (Verbindung Nr. II-2) und 1,2-Dinitro-4-(5'-trifluormethyl-2'-pyridyloxy)-benzol (Verbindung Nr. II-3) eine ausgezeichnete herbizide Wirkung gegen Phaseolus und Solanum :

| Verbindung Nr. | Phaseolus | Solanum |
|---|---|---|
| II-2 | 1 | 1 |
| II-3 | 1 | 3 |

## Patentansprüche

1. Verbindungen der Formel I

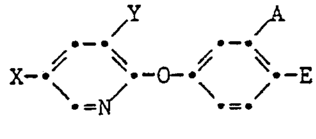

(I)

worin
X Halogen oder Trifluormethyl,
Y Wasserstoff oder Halogen,
einer der beiden Substituenten A und E eine Phosphonatgruppe

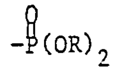

in welcher R für $C_1$-$C_4$-Alkyl oder Wasserstoff steht, und der andere eine Nitrogruppe bedeutet, unter Einschluss ihrer Metallsalze und ihrer Salze mit Ammonium und organischen Stickstoffbasen.

2. Verbindungen der Formel I gemäss Anspruch 1, dadurch gekennzeichnet, dass X für Chlor oder Trifluormethyl, Y für Wasserstoff oder Chlor, einer der Substituenten A und E für die Phosphonatgruppe

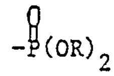

in welcher R Wasserstoff, Methyl oder Aethyl bedeutet, und der andere für die Nitrogruppe steht.

3. Verbindungen der Formel I gemäss Anspruch 1, dadurch gekennzeichnet, dass A für de Phosphonatgruppe

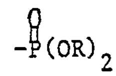

in welcher R Wasserstoff, Methyl oder Aethyl bedeutet, E für die Nitrogruppe steht und X und Y die für Formel I angegebenen Bedeutungen haben.

4. Verbindungen der Formel I gemäss Anspruch 1, dadurch gekennzeichnet, dass A für die Phosphonatgruppe,

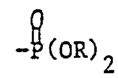

in welcher R Wasserstoff, Methyl oder Aethyl bedeutet, und E für die Nitrogruppe steht, X Chlor oder Trifluormethyl und Y Wasserstoff oder Chlor bedeutet.

5. 2-Nitro-5-(5'-trifluormethyl-2'-pyridyloxy)-phenylphosphonsäure-dimethylester.

6. 2-Nitro-5-(5'-trifluormethyl-2'-pyridyloxy)-phenylphosphonsäure.

7. 2-Nitro-5-(3',5'-dichlor-2'-pyridyloxy)-phenylphosphonsäure-dimethylester.

8. 2-Nitro-5-(3',5'-dichlor-2'-pyridyloxy)-phenylphosphonsäure.

9. 2-Nitro-5-(3'-chlor-5'-trifluormethyl-2'-pyridyloxy)-phenylphosphonsäure-dimethylester.

10. 2-Nitro-5-(3'-chlor-5'-trifluormethyl-2'-pyridyloxy)-phenylphosphonsäure-diäthylester.

11. 2-Nitro-5-(3'-chlor-5'-trifluormethyl-2'-pyridyloxy)-phenylphosphonsäure.

12. 2-Nitro-5-(3',5'-dichlor-2'-pyridyloxy)-phenylphosphonsäure-diäthylester.

13. 2-Nitro-5-(5'-trifluormethyl-2'-pyridyloxy)-phenylphosphonsäure-diäthylester.

14. 2-Nitro-4-(5'-trifluormethyl-2'-pyridyloxy)-phenylphosphonsäure-dimethylester.

15. Verfahren zur Herstellung von Verbindungen der Formel I gemäss Anspruch 1, dadurch gekennzeichnet, dass man eine Verbindung der Formel II

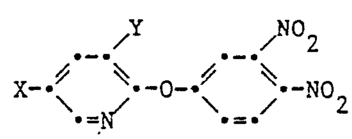

(II)

worin X Halogen oder Trifluormethyl und Y Wasserstoff oder Halogen bedeutet, mit einem Trialkylphosphit der Formel III

$$P(OR')_3 \qquad (III)$$

worin R' für $C_1$-$C_4$-Alkyl steht, umsetzt, und gewünschtenfalls den erhaltenen Phosphonsäuredialkylester der Formel IV

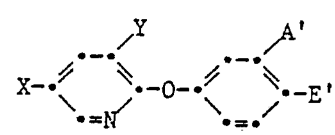

(IV)

worin X und Y die für Formel II angegeben Bedeutungen haben, einer der beiden Substituenten A' und E' eine Phosphonatgruppe

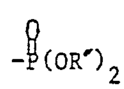

worin R' die vorstehend angegebene Bedeutung hat, und der andere eine Nitrogruppe darstellt, in die freie Phosphonsäure, ein Metallsalz oder ein Salz mit Ammonium oder einer organischen Stickstoffbase überführt.

16. Verfahren nach Anspruch 15, dadurch gekennzeichnet, dass man eine Verbindung der Formel II, welche durch Nitrierung einer Verbindung der Formel V

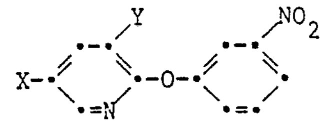

(V)

worin X Halogen oder Trifluormethyl und Y Wasserstoff oder Halogen bedeutet, mit einem Nitriersäuregemisch hergestellt wird, mit einer Verbindung der Formel III umsetzt.

17. Verbindungen der Formel II

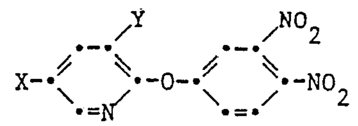

(II)

worin X Halogen oder Trifluormethyl und Y Wasserstoff oder Halogen bedeutet mit der Massgabe, dass X nicht Chlor oder Trifluormethyl bedeutet, wenn Y für Wasserstoff oder Chlor steht.

18. Verfahren zur Herstellung von Verbindungen der Formel II gemäss Anspruch 17, dadurch gekennzeichnet, dass man eine Verbindung der Formel V

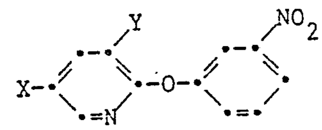

(V)

worin X Halogen oder Trifluormethyl und Y Wasserstoff oder Halogen bedeutet mit der Massgabe, dass X nicht Chlor oder Trifluormethyl bedeutet, wenn Y für Wasserstoff oder Chlor steht, mit einem Nitriersäuregemisch nitriert.

19. Mittel zur Bekämpfung von Unkräutern, dadurch gekennzeichnet, dass es als aktive Komponente eine Verbindung der Formel I gemäss Anspruch 1 enthält.

20. Mittel nach Anspruch 19, dadurch gekennzeichnet, dass es als aktive Komponente 2-Nitro-5-(3'-chlor-5'-trifluormethyl-2'-pyridyloxy)-phenylphosphonsäure-dimethylester enthält.

21. Mittel zur Bekämpfung von Unkräutern, dadurch gekennzeichnet, dass es als aktive Komponente eine Verbindung der Formel II gemäss Anspruch 17 enthält.

22. Mittel zur Regulierung des Pflanzenwachstums, dadurch gekennzeichnet, dass es als aktive Komponente eine Verbindung der Formel I enthält.

23. Mittel nach Anspruch 22 zur Regulierung des Wachstums von Leguminosen.

24. Mittel nach Anspruch 23 zur Regulierung des Wachstums von Sojapflanzen.

25. Mittel nach Anspruch 24, dadurch gekennzeichnet, dass es als aktive Komponente 2-Nitro-5-(5'-trifluormethyl-2'-pyridyloxy)-phenylphosphonsäure-dimethylester enthält.

26. Mittel nach Anspruch 24, dadurch gekennzeichnet, dass es als aktive Komponente 2-Nitro-5-(5'-trifluormethyl-2'-pyridyloxy)-phenylsphosphonsäure enthält.

27. Mittel nach Anspruch 22 zur Förderung des Wurzelwachstums von Kulturpflanzen.

28. Mittel nach Anspruch 27 zur Förderung des Wulzelwachstums von Getreide.

29. Mittel nach Anspruch 28 zur Förderung des Wulzelwachstums von Weizen.

30. Mittel nach Anspruch 27 zur Förderung des Wulzelwachstums von Baumwolle.

31. Mittel nach Anspruch 27, dadurch gekennzeichnet, dass es als aktive Komponente 2-Nitro-5-(5'-trifluormethyl-2'-pyridyloxy)-phenylphosphonsäure enthält.

32. Mittel nach Anspruch 27, dadurch gekennzeichnet, dass es als aktive Komponente 2-Nitro-5-(3', 5'-dichlor-2'-pyridyloxy)-phenylphosphonsäure enthält.

33. Mittel zur Bekämpfung phytopathogener Pilze, dadurch gekennzeichnet, dass es als aktive Komponente eine Verbindung der Formel I gemäss Anspruch 1 enthält.

34. Mittel nach Anspruch 33, dadurch gekennzeichnet, dass es als aktive Komponente 2-Nitro-5-(5'-trifluormethyl-2'-pyridyloxy)-phenylphosphosphonsäure-dimethylester enthält.

35. Mittel nach Anspruch 33, dadurch gekennzeichnet, dass es als aktive Komponente 2-Nitro-5-(5'-trifluormethyl-2'-pyridyloxy)-phenylphosphonsäure enthält.

36. Mittel zur Bekämpfung phytopathogener Bakterien, dadurch gekennzeichnet, dass es als aktive Komponente eine Verbindung der Formel I gemäss Anspruch 1 enthält.

37. Mittel nach Anspruch 36, dadurch gekennzeichnet, dass es als aktive Komponente 2-Nitro-5-(3',5'-dichlor-2'-pyridyloxy)-phenylphosphonsäure-dimethylester enthält.

38. Mittel nach Anspruch 36, dadurch gekennzeichnet, dass es als aktive Komponente 2-Nitro-5-(3'-chlor-5'-trifluormethyl-2'-pyridyloxy)-phenylphosphonsäure-dimethylester enthält.

39. Verfahren zur Bekämpfung von Unkräutern, dadurch gekennzeichnet, dass man eine wirksame Menge eines Wirkstoffs der Formel I gemäss Anspruch 1 auf die Unkräuter, deren Pflanzenteile oder deren Standort appliziert.

40. Verfahren nach Anspruch 41, dadurch gekennzeichnet, dass man 2-Nitro-5-(3′-chlor-5′-trifluormethyl-2′-pyridyloxy)-phenylphosphonsäure-dimethylester appliziert.

41. Verfahren zur Bekämpfung von Unkräutern, dadurch gekennzeichnet, dass man eine wirksame Menge eines Wirkstoffs der Formel II gemäss Anspruch 17 auf die Unkräuter, deren Pflanzenteile oder deren Standort appliziert.

42. Verfahren zur Regulierung des Pflanzenwachstums, dadurch gekennzeichnet, dass man eine wirksame Menge eines Wirkstoffs der Formel I gemäss Anspruch 1 auf die Pflanzen, auf Pflanzenteile oder deren Standort appliziert.

43. Verfahren nach Anspruch 42 zur Regulierung des Wachstums von Kulturpflanzen, dadurch gekennzeichnet, dass man eine wirksame Menge eines Wirkstoffes der Formel I af die Kulturpflanzen appliziert.

44. Verfahren nach Anspruch 43 zur Regulierung des Wachstums von Leguminosen.

45. Verfahren nach Anspruch 44 zur Regulierung des Wachstums von Sojapflanzen.

46. Verfahren nach Anspruch 45, dadurch gekennzeichnet, dass man 2-Nitro-5-(5′-trifluormethyl-2′-pyridyloxy)-phenylphosphonsäure-dimethylester appliziert.

47. Verfahren nach Anspruch 42 zur Förderung des Wurzelwachstums von Kulturpflanzen, dadurch gekennzeichnet, dass man eine wirksame Menge eines Wirkstoffs der Formel I auf die Kulturpflanzen appliziert.

48. Verfahren nach Anspruch 47 zur Förderung des Wurzelwachstums von Getriede.

49. Verfahren nach Anspruch 48 zur Förderung des Wurzelwachstums von Weizen.

50. Verfahren nach Anspruch 47 zur Förderung des Wurzelwachstums von Baumwolle.

51. Verfahren nach Anspruch 47, dadurch gekennzeichnet, dass man 2-Nitro-5-(5′-trifluormethyl-2′-pyridyloxy)-phenylphosphonsäure appliziert.

52. Verfahren nach Anspruch 47, dadurch gekennzeichnet, dass man 2-Nitro-5-(3′,5′-dichlor-2′-pyridyloxy)-phenylphosphonsäure appliziert.

53. Verfahren nach Bekämpfung phytopathogener Pilze, dadurch gekennzeichnet, dass man eine wirksame Menge eines Wirkstoffs der Formel I gemäss Anspruch 1 auf Pflanzen oder Pflanzenteile appliziert.

54. Verfahren nach Anspruch 53, dadurch gekennzeichnet, dass man 2-Nitro-5-(5′-trifluormethyl-2′-pyridyloxy)-phenylphosphonsäure-dimethylester appliziert.

55. Verfahren nach Anspruch 53, dadurch gekennzeichnet, dass man 2-Nitro-5-(5′-trifluormethyl-2′-pyridyloxy)-phenylphosphonsäure appliziert.

56. Verfahren zur Bekämpfung phytopathogener Bakterien, dadurch gekennzeichnet, dass man eine wirksame Menge eines Wirkstoffs der Formel I gemäss Anspruch 1 auf Pflanzen oder Pflanzenteile appliziert.

57. Verfahren nach Anspruch 56, dadurch gekennzeichnet, dass man 2-Nitro-5-(3′,5′-pyridyloxy)-phenylphosphonsäure-dimethylester appliziert.

58. Verfahren nach Anspruch 56, dadurch gekennzeichnet, dass man 2-Nitro-5-(3′-chlor-5′-trifluormethyl-2′-pyridyloxy)-phenylphosphonsäure-dimethylester appliziert.

59. Verwendung einer Verbindung der Formel I gemäss Anspruch 1 oder eines Mittels, welches diese Verbindung enthält, zur Bekämpfung von Unkräutern.

60. Verwendung einer Verbindung der Formel II gemäss Anspruch 17 oder eines Mittels, welches diese Verbindung enthält, zur Bekämpfung von Unkräutern.

61. Verwendung einer Verbindung der Formel I gemäss Anspruch 1 oder eines Mittels, welches diese Verbindung enthält, zur Regulierung des Pflanzenwachstums.

62. Verwendung nach Anspruch 61 zur Steigerung des generativen Wachstums von Kulturpflanzen.

63. Verwendung nach Anspruch 61 zur Förderung des Wurzelwachstums.

## Claims

1. A compound of the formula I

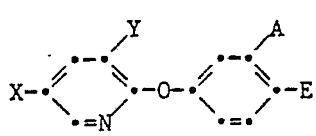

(I)

wherein X is halogen or trifluormethyl, Y is hydrogen or halogen, one of the two substituents A and E is a phosphonate group

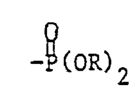

wherein R is $C_1$-$C_4$alkyl or hydrogen and the other is a nitro group, or a metal salt thereof or a salt thereof with ammonium or an organic nitrogen base.

2. A compound of the formula I according to claim 1, wherein X is chlorine or trifluoromethyl, Y is hydrogen or chlorine, one of A and E is the phosphonate group

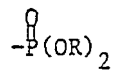

wherein R is hydrogen, methyl or ethyl, and the other is the nitro group.

3. A compound of the formula I according to claim 1, wherein A is the phosphonate group

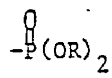

wherein R is hydrogen, methyl or ethyl, E is the nitro group, and X and Y are as defined for formula I.

4. A compound of the formula I according to claim 1, wherein A is the phosphonate group

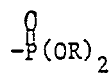

wherein R is hydrogen, methyl or ethyl, E is the nitro group, X is chlorine or trifluoromethyl and Y is hydrogen or chlorine.

5. Dimethyl 2-nitro-5-(5'-trifluoromethyl-2'-pyridyloxy)-phenylphosphonate.

6. 2-Nitro-5-(5'-trifluoromethyl-2'-pyridyloxy)phenylphosphonic acid.

7. Dimethyl 2-nitro-5-(3',5'-dichloro-2'-pyridyloxy)-phenylphosphonate.

8. 2-Nitro-5-(3',5'-dichloro-2'-pyridyloxy)phenylphosphonic acid.

9. Dimethyl 2-nitro-5-(3'-chloro-5'-trifluoromethyl-2'-pyridyloxy)phenylphosphonate.

10. Diethyl 2-nitro-5-(3'-chloro-5'-trifluoromethyl-2'-pyridyloxy)phenylphosphonate.

11. 2-Nitro-5-(3'-chloro-5'-trifluoromethyl-2'-pyridyloxy)-phenylphosphonic acid.

12. Diethyl 2-nitro-5-(3',5'-dichloro-2'-pyridyloxy)phenylphosphonate.

13. Diethyl 2-nitro-5-(5'-trifluoromethyl-2'-pyridyloxy)-phenylphosphonate.

14. Dimethyl 2-nitro-4-(5'-trifluoromethyl-2'-pyridyloxy)-phenylphosphonate.

15. A process for the preparation of a compound of the formula I according to claim 1, which process comprises reacting a compound of the formula II

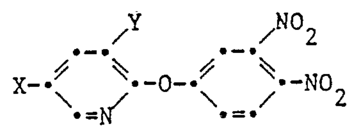

(II)

wherein X is halogen or trifluoromethyl and Y is hydrogen or halogen, with a trialkylphosphite of the formula III

$$P(OR')_3 \qquad \text{(III)}$$

wherein R' is $C_1$-$C_4$alkyl, and, if desired, converting the resultant dialkyl phosphonate of the formula IV

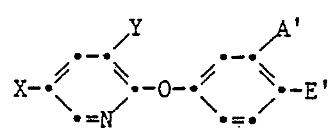

(IV)

wherein X and Y are as defined for formula II, one of the two substituents A' and E' is a phosphonate group

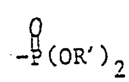

wherein R' is as defined above, and the other is a nitro group, into the corresponding free phosphonic acid, a metal salt or a salt with ammonium or with an organic nitrogen base.

16. A process according to claim 15, which comprises reacting a compound of the formula II, which is prepared by nitration of a compound of the formula V

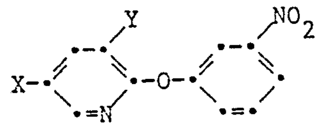

(V)

wherein X is halogen or trifluoromethyl and Y is hydrogen or halogen, with a nitrating acid mixture, with a compound of the formula III.

17. A compound of the formula II

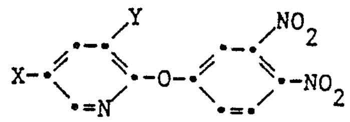

(II)

wherein X is halogen or trifluoromethyl and Y is hydrogen or halogen, with the proviso that X is not chlorine or trifluoromethyl if Y is hydrogen or chlorine.

18. A process for the preparation of a compound of the formula II according to claim 17, which process comprises nitrating a compound of the formula V

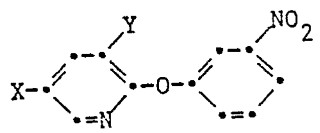

(V)

wherein X is halogen or trifluoromethyl and Y is hydrogen or halogen, with a nitrating acid mixture, with the proviso that X is not chlorine or trifluoromethyl if Y is hydrogen or chlorine.

19. A composition for controlling weeds, which contains a compound of the formula I according to claim 1 as active component.

20. A composition according to claim 19, which contains dimethyl 2-nitro-5-(3'-chloro-5'-trifluoromethyl-2'-pyridyloxy)phenylphosphonate as active component.

21. A composition for controlling weeds, which contains a compound of the formula II according to claim 17 as active component.

22. A composition for regulating plant growth, which contains a compound of the formula I as active component.

23. A composition according to claim 22 for regulating the growth of leguminosae.

24. A composition according to claim 23 for regulating the growth of soya beans.

25. A composition according to claim 24, which contains dimethyl 2-nitro-5-(5'-trifluoromethyl-2'-pyridyloxy)phenylphosphonate as active component.

26. A composition according to claim 24, which contains 2-nitro-5-(5'-trifluoromethyl-2'-pyridyloxy)phenylphosphonic acid as active component.

27. A composition according to claim 22 for stimulating the root growth of cultivated plants.

28. A composition according to claim 27 for stimulating the root growth of cereals.

29. A composition according to claim 28 for stimulating the root growth of wheat.

30. A composition according to claim 27 for stimulating the root growth of cotton plants.

31. A composition according to claim 27, which contains 2-nitro-5-(5'-trifluoromethyl-2'-pyridyloxy)phenylphosphonic acid as active component.

32. A composition according to claim 27, which contains 2-nitro-5-(3',5'-dichloro-2'-pyridyloxy)phenylphosphonic acid as active component.

33. A composition for controlling phytopathogenic fungi, which contains a compound of the formula I according to claim 1 as active component.

34. A composition according to claim 33, which contains dimethyl 2-nitro-5-(5'-trifluoromethyl-2'-pyridyloxy)phenylphosphonate as active component.

35. A composition according to claim 33, which contains 2-nitro-5-(5'-trifluoromethyl-2'-pyridyloxy)phenylphosphonic acid as active component.

36. A composition for controlling phytopathogenic bacteria, which contains a compound of the formula I according to claim 1 as active component.

37. A composition according to claim 36, which contains dimethyl 2-nitro-5-(3',5'-dichloro-2'-pyridyloxy)phenylphosphonate as active component.

38. A composition according to claim 36, which contains dimethyl 2-nitro-5-(3'-chloro-5'-trifluoromethyl-2'-pyridyloxy)phenylphosponate as active component.

39. A method of controlling weeds, which method comprises applying to said weeds, to parts thereof or to the locus thereof an effective amount of a compound of the formula I according to claim 1.

40. A method according to claim 39, wherein dimethyl 2-nitro-5-(3'-chloro-5'-trifluoromethyl-2'-pyridyloxy)-phenylphosphonate is applied.

41. A method of controlling weeds, which comprises applying to said weeds to parts thereof or to the locus thereof, an effective amount of a compound of the formula II according to claim 17.

42. A method of regulating plant growth, which comprises applying to plants,to parts thereof or to the locus thereof, an effective amount of a compound of the formula I according to claim 1.

43. A method according to claim 42 for regulating the growth of cultivated plants, which comprises applying to said plants an effective amount of a compound of the formula I.

44. A method according to claim 43 for regulating the growth of leguminosae.

45. A method according to claim 44 for regulating the growth of soya beans.

46. A method according to claim 45, wherein dimethyl 2-nitro-5-(5'-trifluoromethyl-2'-pyridyloxy) phenylphosphonate is applied.

47. A method according to claim 42 for stimulating the root growth of cultivated plants, which comprises applying to said plants an effective amount of a compound of the formula I according to claim 1.

48. A method according to claim 47 for stimulating the root growth of cereals.

49. A method according to claim 48 for stimulating the root growth of wheat.

50. A method according to claim 47 for stimulating the root growth of cotton plants.

51. A method according to claim 47, wherein 2-nitro-5-(5'-trifluoromethyl-2'-pyridyloxy) phenylphosphonic acid is applied.

52. A method according to claim 47, wherein 2-nitro-5-(3',5'-dichloro-2'-pyridyloxy) phenylphosphonic acid is applied.

53. A method of controlling phytopathogenic fungi, which comprises applying to plants or to parts thereof an effective amount of a compound of the formula I according to claim 1.

54. A method according to claim 53, wherein dimethyl 2-nitro-5-(5'-trifluoromethyl-2'-pyridyloxy) phenylphosphonate is applied.

55. A method according to claim 53, wherein 2-nitro-5-(5'-trifluoromethyl-2'-pyridyloxy) phenylphosphonic acid is applied.

56. A method of controlling phytopathogenic bacteria, which comprises applying to plants or to parts thereof an effective amount of a compound of the formula I according to claim 1.

57. A method according to claim 56, wherein dimethyl 2-nitro-5-(3',5'-dichloro-2'-pyridyloxy) phenylphosphonate is applied.

58. A method according to claim 56, wherein dimethyl 2-nitro-5-(3'-chloro-5'-trifluoromethyl-2'-pyridyloxy) phenylphosphonate is applied.

59. Use of a compound of the formula I according to claim 1 or of a composition containing said compound, for controlling weeds.

60. Use of a compound of the formula II according to claim 17, or of a composition containing said compound, for controlling weeds.

61. Use of a compound of the formula I according to claim 1, or of a composition containing said compound, for regulating plant growth.

62. Use according to claim 61 for increasing the vegetative growth of plants.

63. Use according to claim 61 for promoting root growth.

**Revendications**

1. Composés de formule I

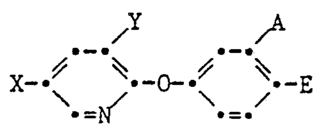

(I)

où X représente un halogène ou un trifluorométhyle, Y représente un hydrogène ou un halogène, l'un des deux substituants A et E un groupe phosphonate

$$-\overset{O}{\overset{\|}{P}}(OR)_2$$

où R représente un alcoyle en C1 à C4 ou un hydrogène, et l'autre représente un groupe nitro, y compris leurs sels métalliques et leurs sels avec l'ammonium et les bases azotées organiques.

2. Composés de formule I selon la revendication 1, caractérisés en ce que X représente un chlore ou un trifluorométhyle, Y représente un hydrogène ou un chlore, l'un des substituants A et E représente le groupe phosphonate

$$-\overset{O}{\overset{\|}{P}}(OR)_2$$

où R représente un hydrogène, un méthyle ou un éthyle, et l'autre représente le groupe nitro.

3. Composés de formule I selon la revendication 1, caractérisés en ce que A représente le groupe phosphonate

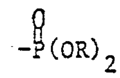

où R représente un hydrogène, un méthyle ou un éthyle, E représente le groupe nitro, et X et Y ont les significations données pour la formule I.

4. Composés de formule I selon la revendication 1, caractérisés en ce que A représente le groupe phosphonate

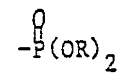

où R représente un hydrogène, un méthyle ou un éthyle, E représente le groupe nitro, et X représente un chlore ou un trifluorométhyle et Y représente un hydrogène ou un chlore.

5. Diméthylester de l'acide 2-nitro-5-(5'-trifluorométhyl-2'-pyridyloxy)-phénylphosphonique ;

6. Acide 2-nitro-5-(5'-trifluorométhyl-2'-pyridyloxy)-phénylphosphonique ;

7. Diméthylester de l'acide 2-nitro-5-(3',5'-dichloro-2'-pyridyloxy)-phénylphosphonique ;

8. Acide 2-nitro-5-(3',5'-dichloro-2'-pyridyloxy)-phénylphosphonique ;

9. Diméthylester de l'acide 2-nitro-5-(3'-chloro-5'-trifluorométhyl-2'-pyridyloxy)-phénylphosphonique ;

10. Diéthylester de l'acide 2-nitro-5-(3'-chloro-5'-trifluorométhyl-2'-pyridyloxy)-phénylphosphonique ;

11. Acide 2-nitro-5-(3'-chloro-5'-trifluorométhyl-2'-pyridyloxy)-phénylphosphonique ;

12. Diéthylester de l'acide 2-nitro-5-(3',5'-dichloro-2'-pyridyloxy)-phénylphosphonique ;

13. Diéthylester de l'acide 2-nitro-5-(5'-trifluorométhyl-2'-pyridyloxy)-phénylphosphonique ;

14. Diméthylester de l'acide 2-nitro-4-(5'-trifluorométhyl-2'-pyridyloxy)-phénylphosphonique.

15. Procédé de préparation de composés de formule I selon la revendication 1, caractérisé en ce que l'on fait réagir un composé de formule II

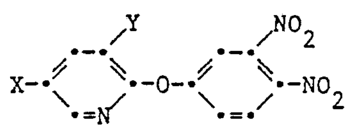

(II)

où X représente un halogène ou un trifluorométhyle et Y représente un hydrogène ou un halogène, avec un trialcoylphosphite de formule III

$$P(OR')_3 \quad (III),$$

où R' représente un alcoyle en C1 à C4, et si on le désire on transforme le dialcoylester d'acide phosphonique obtenu de formule IV

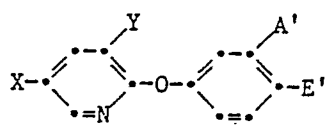

(IV)

où X et Y ont les significations données pour la formule II, l'un des deux substituants A' et E' représente un groupe phosphonate

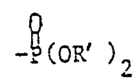

où R' a la signification donnée ci-dessus, et l'autre représente un groupe nitro, de façon connue pour donner l'acide phosphonique libre correspondant, un sel métallique ou un sel avec l'ammonium ou une base azotée organique.

16. Procédé selon la revendication 15, caractérisé en ce qu'on prépare un fait réagir un composé de formule II, que l'on prépare par nitration d'un composé de formule V

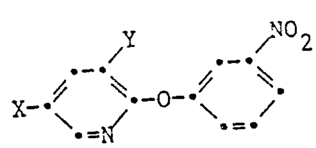

(V)

où X représente un halogène ou un trifluorométhyle et Y représente un hydrogène ou un halogène, avec un mélange sulfo-nitrique, avec un composé de formule III.

17. Composés de formule II

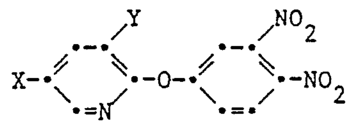

(II)

où X représente un halogène ou un trifluorométhyle et Y représente un hydrogène ou un halogène, avec la précision que X ne représente pas un chlore ou un trifluorométhyle lorsque Y représente un hydrogène ou un chlore.

18. Procédé de préparation de composés de formule II selon la revendication 17, caractérisé en ce qu'on nitre avec un mélange sulfo-nitrique un composé de formule V

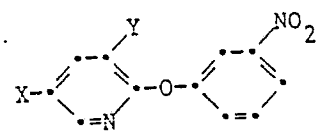

(V)

où X représente un halogène ou un trifluorométhyle et Y représente un hydrogène ou un halogène, avec la précision que X ne représente pas un chlore ou un trifluorométhyle lorsque Y représente un hydrogène ou un chlore.

19. Agent pour lutter contre les mauvaises herbes, caractérisé en ce qu'il contient comme composant actif un composé de formule I selon la revendication 1.

20. Agent selon la revendication 19, caractérisé en ce qu'il contient comme composant actif le diméthylester de l'acide 2-nitro-5-(3'-chloro-5'-trifluorométhyl-2'-pyridyloxy)-phénylphosphonique.

21. Agent pour combattre les mauvaises herbes, caractérisé en ce qu'il contient comme composant actif un composé de formule II selon la revendication 17.

22. Agent de régulation de la croissance des plantes, caractérisé en ce qu'il contient comme composant actif un composé de formule I.

23. Agent selon la revendication 22 de régulation de la croissance des légumineuses.

24. Agent selon la revendication 23 de régulation de la croissance des plants de soja.

25. Agent selon la revendication 24, caractérisé en ce qu'il contient comme composant actif le diméthylester de l'acide 2-nitro-5-(5'-trifluorométhyl-2'-pyridyloxy)-phénylphosphonique.

26. Agent selon la revendication 24, caractérisé en ce qu'il contient comme composant actif l'acide 2-nitro-5-(5'-trifluorométhyl-2'-pyridyloxy)-phénylphosphonique.

27. Agent selon la revendication 22 pour faciliter la croissance des racines des plantes cultivées.

28. Agent selon la revendication 27 pour faciliter la croissance des racines des céréales.

29. Agent selon la revendication 28 pour faciliter la croissance des racines du blé.

30. Agent selon la revendication 27 pour faciliter la croissance des racines du coton.

31. Agent selon la revendication 27, caractérisé en ce qu'il contient comme composant actif l'acide 2-nitro-5-(5'-trifluorométhyl-2'-pyridyloxy-phénylphosphonique.

32. Agent selon la revendication 27, caractérisé en ce qu'il contient comme composant actif l'acide 2-nitro-5-(3',5'-dichloro-2'-pyridyloxy-)-phénylphosphonique.

33. Agent pour combattre les champignons phytopathogènes, caractérisé en ce qu'il contient comme composant actif un composé de formule I selon la revendication 1.

34. Agent selon la revendication 33, caractérisé en ce qu'il contient comme composant actif le diméthylester de l'acide 2-nitro-5-(5'-trifluorométhyl-2'-pyridyloxy)-phénylphosphonique.

35. Agent selon la revendication 33, caractérisé en ce qu'il contient comme composant actif l'acide 2-nitro-5-(5'-trifluorométhyl-2'-pyridyloxy)-phénylphosphonique.

36. Agent pour combattre les bactéries phytopathogènes, caractérisé en ce qu'il contient comme composant actif un composé de formule I selon la revendication 1.

37. Agent selon la revendication 36, caractérisé en ce qu'il contient comme composant actif le diméthylester de l'acide 2-nitro-5-(3',5'-dichloro-2'-pyridyloxy)-phénylphosphonique.

38. Agent selon la revendication 36, caractérisé en ce qu'il contient comme composant actif le diméthylester de l'acide 2-nitro-5-(3'-chloro-5'-trifluorométhyl-2'-pyridyloxy)-phénylphosphonique.

39. Procédé de lutte contre les mauvaises herbes, caractérisé en ce qu'on applique une quantité efficace d'une matière active de formule I selon la revendication 1 aux mauvaises herbes, aux parties de ces plantes ou à l'endroit où elles poussent.

40. Procédé selon la revendication 41, caractérisé en ce qu'on applique le diméthylester de l'acide 2-nitro-5-(3'-chloro-5'-trifluorométhyl-2'-pyridyloxy)-phénylphosphonique.

41. Procédé de lutte contre les mauvaises herbes, caractérisé en ce qu'on applique une quantité efficace d'une matière active de formule II selon la revendication 17 aux mauvaises herbes, aux parties de ces plantes ou à l'endroit où elles poussent.

42. Procédé de régulation de la croissance des plantes, caractérisé en ce qu'on applique une quantité efficace d'une matière active de formule I selon la revendication 1 aux plantes, aux parties des plantes ou à l'endroit où elles poussent.

43. Procédé selon la revendication 42 de régulation de la croissance des plantes cultivées, caractérisé en ce qu'on applique une quantité efficace d'une matière active de formule I aux plantes cultivées.

44. Procédé selon la revendication 43 de régulation de la croissance des légumineuses.

45. Procédé selon la revendication 44 de régulation de la croissance des plants de soja.

46. Procédé selon la revendication 45, caractérisé en ce qu'on aplique le diméthylester de l'acide 2-nitro-5-(5'-trifluorométhyl-2'-pyridyloxy)-phénylphosphonique.

47. Procédé selon la revendication 42 de facilitation de la croissance des racines de plantes cultivées, caractérisé en ce qu'on applique une quantité efficace d'une matière active de formule I aux plantes cultivées.

48. Procédé selon la revendication 47 de facilitation de la croissance des racines de céréales.

49. Procédé selon la revendication 48 de facilitation de la croissance des racines du blé.

50. Procédé selon la revendication 47 de facilitation de la croissance des racines du coton.

51. Procédé selon la revendication 47, caractérisé en ce qu'on applique l'acide 2-nitro-5-(5'-trifluorométhyl-2'-pyridyloxy)-phénylphosphonique.

52. Procédé selon la revendication 47, caractérisé en ce qu'on applique l'acide 2-nitro-5-(3',5'-dichloro-2'-pyridyloxy)-phénylphosphonique.

53. Procédé de lutte contre les champignons phytopathogènes, caractérisé en ce qu'on applique une quantité efficace d'une matière active de formule I selon la revendication 1 aux plantes ou parties de plantes.

54. Procédé selon la revendication 53, caractérisé en ce qu'on applique le diméthylester de l'acide 2-nitro-5-(5'-trifluorométhyl-2'-pyridyloxy)-phénylphosphonique.

55. Procédé selon la revendication 53, caractérisé en ce qu'on applique l'acide 2-nitro-5-(5'-trifluorométhyl-2'-pyridyloxy)-phénylphosphonique.

56. Procédé de lutte contre les bactéries phytopathogènes, caractérisé en ce qu'on applique une quantité efficace d'une matière active de formule I selon la revendication 1 sur les plantes ou parties de plantes.

57. Procédé selon la revendication 56, caractérisé en ce qu'on applique le diméthylester de l'acide 2-nitro-5-(3',5'-dichloro-2'-pyridyloxy)-phénylphosphonique.

58. Procédé selon la revendication 56, caractérisé en ce qu'on applique le diméthylester de l'acide 2-nitro-5-(3'-chloro-5'-trifluorométhyl-2'-pyridyloxy)-phénylphosphonique.

59. Application d'un composé de formule I selon la revendication 1 ou d'un agent qui contient ce composé à la lutte contre les mauvaises herbes.

60. Application d'un composé de formule II selon la revendication 17 ou d'un agent qui contient ce composé pour lutter contre les mauvaises herbes.

61. Application d'un composé de formule I selon la revendication 1 ou d'un agent qui contient ce composé à la régulation de la croissance des plantes.

62. Application selon la revendication 61 à l'accroissement de la croissance générative des plantes cultivées.

63. Application selon la revendication 61 à la facilitation de la croissance des racines.